# EUROPEAN PATENT SPECIFICATION

(11) **EP 1 408 888 B1**
(45) Date of publication and mention of the grant of the patent: **24.03.2010**
(21) Application number: 01955877.4
(22) Date of filing: 20.07.2001
(51) Int. Cl.: A61F 2/44, A61F 2/46

(54) **AN EXPANDABLE POROUS MESH BAG DEVICE AND ITS USE FOR BONE SURGERY**
EINE DEHNBARE, PORÖSE NETZBEUTEL-VORRICHTUNG UND SEINE NUTZUNG IN DER KNOCHENCHIRUGIE
DISPOSITIF DE SAC MAILLE POREUX EXPANSIBLE ET SON UTILISATION POUR LA CHIRURGIE OSSEUSE

(43) Date of publication of application: 21.04.2004
(60) Divisional application: 09009218.0
(73) Proprietor: The Spineology Group, LLC, St. Paul, MN 55128-5451 (US)
(72) Inventor: KUSLICH, Stephen, D., Stillwater, MN 55082 (US); GROBLER, Leon, J., Phoenix, AZ 85029 (US); WOLFE, Steven, J., Cottage Grove, MN 55016 (US); AHERN, James, W., Hopkins, MN 55343 (US)
(74) Representative: Rees, David Christopher
(86) International application number: PCT/US2001/022838
(87) International publication number: WO 2003/007853

(56) References cited:
- US-A- 4 969 888
- US-A- 5 108 404
- US-A- 5 549 679
- US-A- 5 571 189
- US-A- 5 755 797
- US-A- 5 972 015

## Description

This invention relates to a vertebral compression fracture correcting system.

U.S. Patents 5,549,679 and 5,571,189 to Kuslich describe a device and method for stabilizing the spinal segment with an expendable, porous fabric implant for insertion into the interior of a reamed out disc which is packed with material to facilitate bony fusion. In the present invention, a similar bag is used to address compression fractures of the spine.

U.S. Patents 5,108,404 and 4.969,888 to Scholten et al. describe a system for fixing osteoporotic bone using an inflatable balloon which compacts the bone to form a cavity into which bone cement is injected after the balloon is withdrawn. The invention requires the use of fluoroscopy to monitor the injection and to help guard against cement leakage through fissures in bone. Unfortunately, such a leakage is known to occur in spite of these precautions. Since such a leakage may cause serious injury, including paralysis, an improved device and method is needed.

U.S. Patent 5,972,015 to Scribner et al. describes a system of deploying a catheter tube into the interior of a vertebra and expanding a specially configured non-porous balloon therewithin to compact cancellous bone to form a cavity. The Scribner 5,972,015 approach utilizes a non-porous balloon which is inflated within the bone to cause compression. The cavity thus formed may then be filled with bone cement.

Unfortunately, the bag used by Scribner may be ruptured during expansion to compact cancellous bone due to sharp projections found within the cavity to be expanded, resulting in leakage of the inflation medium. Also, filling the cavity eventually formed could allow leakage of bone cement out of the bone against vessels or nerves which may cause undesirable complications.

The present invention involves an improvement of all of the previous systems and avoids complications that could occur with the system of U.S. 5,972.015.

According to the invention, there is provided a vertebral compression fracture correcting system comprising: means for creating a cavity within a bone; an expandable porous bag; and a fill tool constructed to deliver bone filler material under pressure into the porous bag; characterized by: the means for creating the cavity being a reamer arranged to form a cavity within a vertebra; means for inserting the expandable porous bag into the cavity within the vertebra; and the fill tool being arranged to inject bone filler material under pressure into the porous bag, when in place in the cavity within the vertebra, to expand the expandable porous bag to the limits of the cavity.

Thus, the invention enables a method of correcting compression fractures of the spine by first accessing and boring into the damaged tissue or bone and reaming out the damaged and/or diseased area using any of the presently accepted procedures or the damaged area may be prepared by expanding a bag within the damaged tissue the bone must be stabilized.

The reamer may be any conventional reamer. Examples of reamers are described in U.S. Pat. No. 5,415,255. Other examples of reamers are known and may be used. After the damaged bone or tissue has been removed by the reamer, bone repair medium can be inserted into the cavity thus formed, via a catheter and expandable fabric bag as described in U.S. patents 5,549,679 and 5,571,189.

A detailed description of the invention is hereafter described with specific reference being made to the drawings in which:
FIG. 1 is a side elevational view of a vertebra that is fractured and in need of repair;
FIG. 2 is a side view of the vertebra of Fig. 1 being reamed out with a reaming tool from the anterior approach;
FIG. 3 is a top view of the vertebra of Fig. 1 showing the reamer forming a pair of cavities within the vertebra from the anterior approach;
FIG. 4 is a side elevational view of the vertebra of Fig. 2 showing placement of an expandable fabric bag of the invention;
FIG. 5 is a top elevational view of the vertebra of Fig. 3 showing a second of two expandable fabric bags of the invention being positioned;
FIG. 6 is a side view of a vertebra being reamed from a posterior approach;
FIG. 7 is a top view of the vertebra of Fig. 6 with a bag in place and a second cavity being reamed;
FIG. 8 is a side elevational view of the vertebra of Fig. 6 with an expandable fabric bag of the invention in place; and
FIG. 9 is a top view of the vertebra of Fig. 7 with one bag inflated and the second bag being deployed;
In the following detailed description similar reference numerals are used to depict like elements in the various figures.

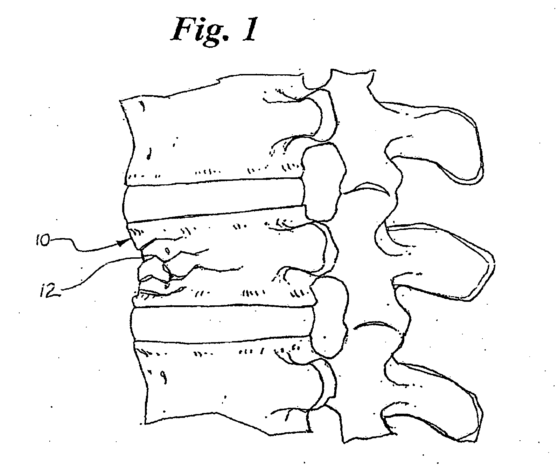

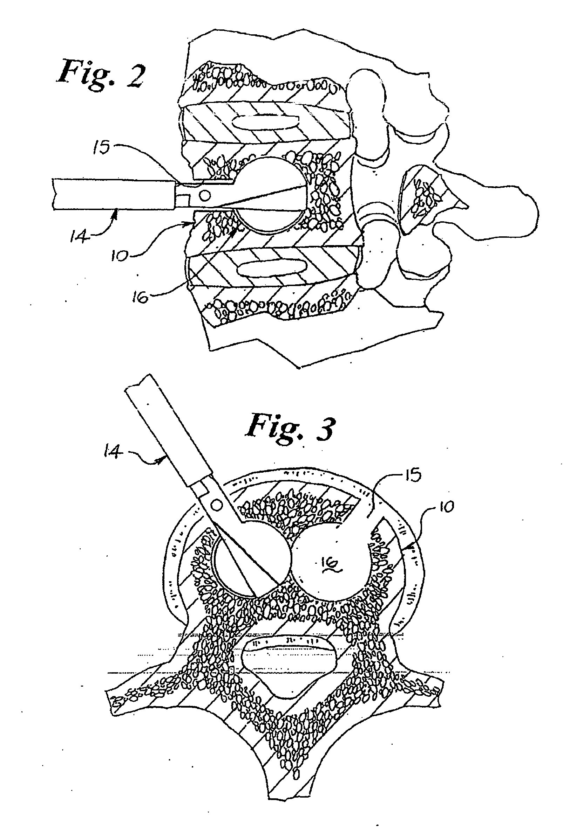

FIG. 1 shows a typical vertebra 10 having compression fractures 12 that is in need of repair. As indicated above the damaged portion of the vertebra 10 is reamed out. For example, FIG. 2 shows a reamer 14 entering the vertebra 10 anteriorly to make an opening 15 and cavity 16. Alternatively, multiple cavities 16 may be formed such as is shown in FIG. 3.

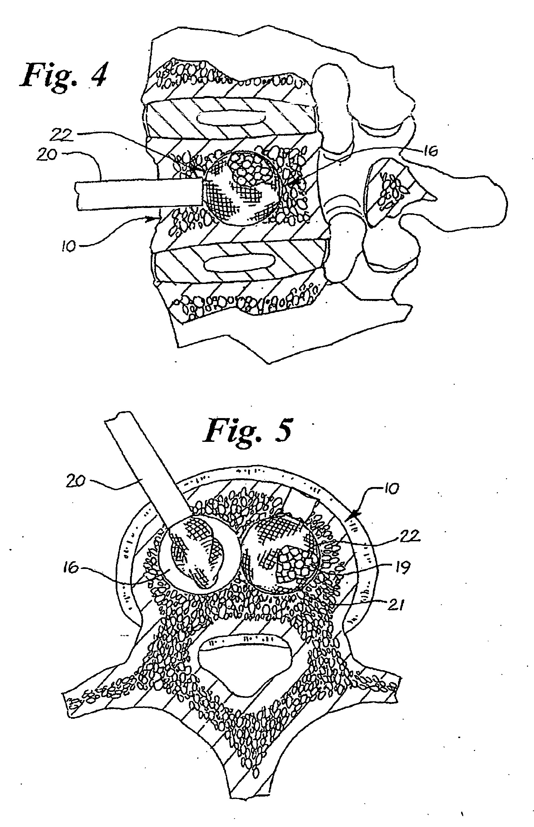

In FIG. 4 a delivery tube or catheter 20 is seen in the process of delivering an expandable fabric bag 22 into the vertebra 10 or into a cavity 16 present therein. Once the bag 22 is positioned within the vertebra 10, the bag 22 may be inflated or expanded to the limits of the cavity 16 thus formed through insertion or injection of fill material 19 into the interior 21 of the bag 22.

FIG. 5 shows a single filled expandable fabric bag 22 in place with a second expandable bag which is being inserted and expanded within the cavity 16.

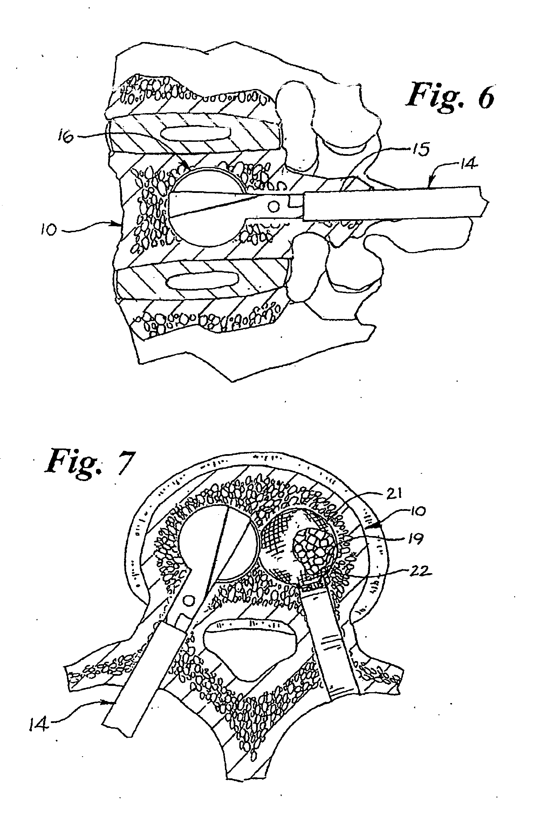

FIGs. 6-9 illustrate a procedure in which the opening 15 and cavity 16 are created posteriorly. Regardless of the direction through which the vertebra 10 is operated on, in all forms, the cavity 16 which is formed is then filled with acceptable bone replacement material.

Bone replacement material 19 may be one or more of the following, or any other biocompatible material judged to have the desired physiologic response:
A) Demineralized bone material, morselized bone graft, cortical, cancellous, or cortico-cancellous, including autograft, allograft, or xenograft.
B) Any bone graft substitute or combination of bone graft substitues, or combinations of bone graft and bone graft substitutes, or bone inducing substances, including but not limited to: Tricalcium phosphates, Tricalcium sulfates, Tricalcium carbonates, hydroxyapatite, bone morphogenic protein, calcified and/or decalcified bone derivative.
C) Bone cements, such as ceramic and polymethylmethacrylate bone cements.

The bone replacement material is inserted into the bag 22 via a needle, catheter 20 or other type of fill tool. The bone replacement material expands the bag to the limits of the cavity 16.

The bag 22 may be a small fabric bag, about one to about four cm in diameter, being roughly spherical in shape, although other elliptical shapes and other geometric shapes may be used. The bag is pliable and malleable before its interior space 21 is filled with the contents to be described. The material of the bag 22 may be configured to take on the shape of the cavity in which the bag is placed. While in this initial condition, the bag may passed, uninflated, though a relatively small tube or portal, perhaps about three mm to about one cm in diameter.

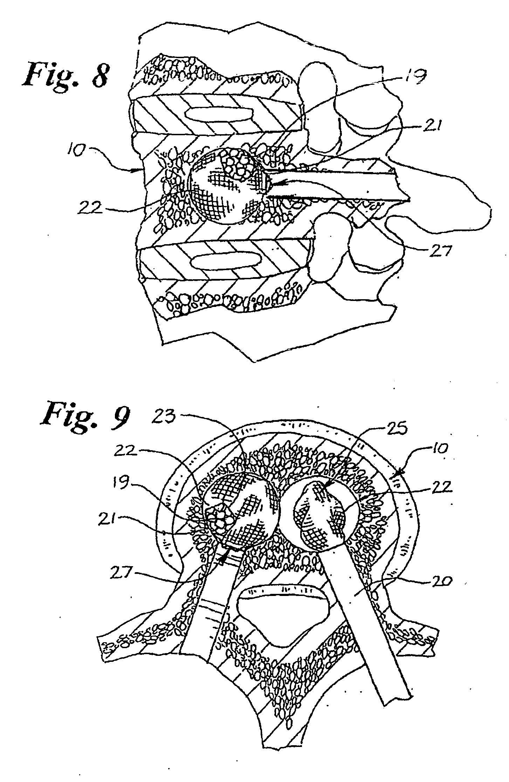

The bag 22 such as may best be seen in FIG. 9, is constructed in a special and novel way. The bag 22 may be constructed of a fabric 23. Fabric 23 may be woven, knitted, braided or form-molded to a density that will allow ingress and egress of fluids and solutions and will allow the ingrowth and through-growth of blood vessels and fibrous tissue and bony trabeculae, but the fabric porosity is tight enough to retain small particles of enclosed material, such as ground up bone graft, or bone graft substitute such as hydroxyapatite or other osteoconductive biocompatible materials known to promote bone formation. The fabric 23 defines a plurality of pores 25. Generally, the pores 25 of the fabric 23 will have a diameter of about 0.25 mm or less, to about 5.0 mm. The size is selected to allow tissue ingrowth while containing the material packed into the bag. If bone cement or other material is used which will not experience bone ingrowth, the pores 25 may be much tighter to prevent egress of the media from within the bag 22 out into the cavity 16. This prevents leakage that could impinge upon nerves, blood vessels or the like if allowed to exit the bone.

One or more of the pores 25 may be used as a fill opening 27, wherein the fabric 23 may be manipulated to enlarge a pore to a diameter potentially greater than 5 mm but no more than about 1 cm. Preferably the fill opening 27 is less than about 5mm in diameter. Such pore/fill opening 27 is sufficiently large to allow a catheter, needle, fill tube or other device for inserting or injecting fill material to pass through the fabric 23 and into the interior 21 of the bag 22 without damaging the integrity of the bag 22.

When the bag 22 is fully filled with fill material, the bag will form a self-retaining shape which substantially fills the cavity 16. Once sufficiently full, the fill tool used to place fill material into the bag interior 21 is removed from the opening 27. Where the opening 27 is not a pore 25 but rather a separate and distinct opening in the bag 22, the opening 27 may have a set diameter which requires sealing such as by tying, fastening, welding, gluing or other means of closing the opening 27 after the bag has been filled. Where the opening 27 is a pore 25, upon removal of the catheter or fill tool from the opening 27 the fabric 23 will contract to reduce the diameter of the opening 27 to be substantially similar to that of the other pores.

The size and density of the pores determine the ease or difficulty with which materials may pass through the mesh. For instance, very small pores (<0.5 mm would prohibit passage of all but the smallest particles and liquid. The pore size and density could be controlled in the manufacturing process, such that the final product would be matched to the needs of the surgeon. For example, if methylmethacrylate bone cement were to used, the pore size would need to be very small such as about less tan 0.5 mm to about 1.0 mm, whereas, when bone graft or biocompatible ceramic granules are used, pore sizes ranging from about 1.0 mm to about 5.0 mm or more may be allowed. The fact that the fabric 16 is properly porous would allow it to restrict potentially dangerous flow of the fill material outside the confines of the bag.

The fabric is light, biocompatible, flexible and easily handled, and has very good tensile strength, and this is unlikely to rip or tear during insertion and inflation. When the device is inflated, the device expands to fill a previously excavated cavity 16.

The use of the term "fabric" herein is meant to include the usual definition of that term and to include any material that functions like a fabric, that is, the "fabric" of the invention must have a plurality of pores 25, through which material and fluid flow is allowed under the terms as described, and the "fabric" must be flexible enough to allow it to be collapsed and inserted into an opening smaller than the inflated bag size.

The bag 22 need not be woven and may be molded or otherwise formed as is well know in the art. The preferred material may provide the ability to tailor bioabsorbance rates. Any suture-type material used medically may be used to form the bag 22. The bag may be formed of plastic or even metal. In at least one embodiment bag 22 is formed using a combination of resorbable and/or nonresorbable thread. Bag 22 may include a fill opening 27 which may be a bushing that could be a bioabsorbable and/or nonbioabsorbable plastic, ceramic or metal. The opening 27 may also be such as hydroxyapatite or it could be plastic it metal. The opening 27 may also be characterized as a pore 25, wherein a pore 25 of the fabric 23 has been expanded to allow a catheter 20 or other fill device to pass into the interior 21 of the bag 22. The bag 22 could be formed from a solid material to which perforations are added. The bag 22 may be partially or totally absorbable, metal, plastic, woven, solid, film or an extruded balloon.

The system of the present invention may be used to treat a damaged vertebra 10, in accordance with the following procedures depicted in FIGs. 1-9:

Initially, the vertebra 10 needing repair is surgically exposed by forming at least one cavity 16. The cavity or cavities 16 are formed by reaming. Reaming may be accomplished by several means such as the use of a reamer 14 as shown for example in U.S. Pat. No. 5,015,255. Next, the unexpanded mesh bag or Expandable Fabric Bag Device (EFBD) 22 is inserted into the cavity or cavities via catheter 20 or other means. At some point, the fill material 19 is prepared for insertion or injection into the EFBD 22. Following preparation of the fill material 19, the material is injected or otherwise inserted into the bag 22 using sufficient pressure to fill the bag 22 to its expanded state, thus producing rigidity and tension within the cavity or cavities 16 to reach the degree of correction required by virtue of the compression fractures. Finally, the fill opening 27 is close to prevent egress of inflation material 19.

## Claims

1. A vertebral compression fracture correcting system comprising: means (14) for creating a cavity (16) within a bone (10), an expandable porous bag, and a fill tool (20) constructed to deliver bone filler material (19) under pressure into the porous bag; **characterized by**: the means (14) for creating the cavity (16) being a reamer arranged to form a cavity within a vertebra (10); means for inserting the expandable porous bag (22) into the cavity (16) within the vertebra (10); and the fill tool (20) being arranged to inject bone filler material (19) under pressure into the porous bag (22), when in place in the cavity (16) within the vertebra, to expand the expandable porous bag (22) to the limits of the cavity (16).

## Patentansprüche

1. System zur Korrektur von Wirbelkompressionsfrakturen, umfassend: Mittel (14) zum Schaffen eines Hohlraums (16) innerhalb eines Knochens (10), einen expandierbaren porösen Beutel (22), und ein Füllwerkzeug (20), das ausgebildet ist, um Knochenfüllmaterial (19) unter Druck in den porösen Beutel (22) abzugeben; **dadurch gekennzeichnet, dass** das Mittel (14) zum Bilden des Hohlraums (16), ein Räumwerkzeug ist, welches eingerichtet ist, einen Hohlraum (16) innerhalb eines Wirbels (10) zu bilden; durch Mittel zum Einsetzen des expandierbaren porösen Beutels (22) in den Hohlraum (16) innerhalb des Wirbels (10); und dass das Füllwerkzeug (20), eingerichtet ist, Knochenfüllmaterial (19) unter Druck in den porösen Beutel (22) zu injizieren, wenn er im Hohlraum (16) innerhalb des Wirbels (10) positioniert ist, um den expandierbaren porösen Beutel (22) bis an die Grenzen des Hohlraums (16) zu expandieren.

## Revendications

1. Système de correction d'une fracture vertébrale par compression comprenant : un moyen (14) pour créer une cavité (16) dans un os (10), une sac poreux expansible, et un outil de remplissage (20) conçu pour délivrer un matériau de remplissage osseux (19) sous pression dans le sac poreux ; **caractérisé par** : le moyen (14) pour créer la cavité (16) étant un alésoire conçu pour former une cavité dans une vertèbre (10) ; un moyen pour insérer le sac poreux expansible (22) dans la cavité (16) dans la vertèbre (10) ; et l'outil de remplissage (20) étant conçu pour injecter le matériau de remplissage osseux (19) sous pression dans le sac poreux (22), lorsque placé dans la cavité (16) dans la vertèbre, pour étendre le sac poreux expansible (22) aux limites de la cavité (16).
